# EUROPEAN PATENT SPECIFICATION

(11) **EP 4 216 220 B1**
(45) Date of publication and mention of the grant of the patent: **24.06.2026**
(21) Application number: 22191690.1
(22) Date of filing: 23.08.2022
(51) Int. Cl.: G11C 13/00, G11C 13/04, C12Q 1/68

(54) **DATA STORAGE DEVICE AND METHOD FOR STORING DATA**
DATENSPEICHERVORRICHTUNG UND VERFAHREN ZUM SPEICHERN VON DATEN
DISPOSITIF DE STOCKAGE DE DONNÉES ET PROCÉDÉ DE STOCKAGE DE DONNÉES

(30) Priority: 25.01.2022 EP 22153210
(43) Date of publication of application: 26.07.2023
(73) Proprietor: Leica Microsystems CMS GmbH, 35578 Wetzlar (DE)
(72) Inventor: Alsheimer, Soeren, 60320 Frankfurt (DE); Bradl, Joachim, 68519 Viernheim (DE)
(74) Representative: Schaumburg und Partner Patentanwälte mbB

(56) References cited:
- WO-A1-2020/243187
- US-A1- 2010 262 374
- US-A1- 2018 142 286
- US-A1- 2020 025 752
- US-B1- 6 329 139

## Description

### Technical field:

The present invention relates to a data storage device comprising an oligonucleotide nanostructure backbone and a method for storing and accessing data.

### Background:

DNA, a naturally occurring organic information storage molecule, is stable over hundreds to thousands of years due to its inherent chemical stability, even under unfavourable conditions. In the art, the use of DNA for direct writing of information into the DNA sequences and the stability of DNA written sequences has been established using accelerated aging tests performed at 65°C for 20 days, which corresponds roughly to 20 years at -20°C. These tests found that the original message was maintained without errors (https:!/www.mdpi.com/2073-4360/10/1/28).

Presently available commercially available data storage technologies allow densities in the range of 1-5Tbit/inch². Experimental technologies have been shown that allow -10 Tbit/inch² corresponding to 125G byte and 3nm features using a bottom-up synthesis approach using block-copolymers (https://www.science.org/doi/10.1126/science.1168108). However, with an increase in density the durability of storage media often decreases.

Documents WO 2020/243187 A1, US 2018/142286 A1 and US 2018/262374 A1 discloses methods for sequencing a DNA by binding short optically detectable oligonucleotide probes to the DNA. In each of these documents, an oligonucleotide nanostructure backbone comprising labels is disclosed.

### Summary

It is an object to provide a data storage device and a method that enable high-density, long-term storage of data.

The aforementioned object is achieved by the subject-matter of the independent claims. Advantageous embodiments are defined in the dependent claims and the following description.

Recently, DNA origami technology has opened the possibility to design arbitrary nanostructures based on DNA scaffolds and DNA staple strands. Importantly, these base structures can be used to place labels with nanometer precision in 3D using position-specific staple strands, which may be connected either directly or indirectly to a label. The present invention takes advantage of these disparate technologies to solve the problem of high-density long-term - preferably encrypted - data storage based on widely available and inexpensive substrates.

In one aspect an organic data storage device is provided, comprising: an oligonucleotide nanostructure backbone with a plurality of attachment sites at predetermined positions; a plurality of labels configured to attach to some or all of the attachment sites; at least a first orientation indicator and a second orientation indicator; wherein each label comprises at least one dye, and an attachment oligonucleotide portion configured to attach the label to one of the attachment sites; and wherein the attachment oligonucleotide portion of each label comprises a unique oligonucleotide sequence configured to - preferably reversibly - bind to a complementary sequence of one of the attachment sites.

By providing or generating the oligonucleotides nanostructure backbone in a particular or predetermined manner, the position of the attachment sites for the labels relative to the oligonucleotide nanostructure backbone and/or the at least one first orientation indicator and one second orientation indicator are predetermined or known. Thus, providing a suitable or predetermined plurality of labels to attach at the corresponding attachment sites, information can be stored at the data storage device.

An oligonucleotide is, for example, a single stranded DNA or RNA molecule, that may be sequenced to determine its sequence of nucleotides. Complementary parts of oligonucleotides may hybridise or bind to each other.

The orientation indicators are configured to attach to the backbone, and may be used to visually determine the orientation of the data storage device in space.

Preferably, the nanostructure backbone comprises scaffold strands, and staple strands configured to bind to the scaffold strands at predetermined positions to fold the scaffold strand into a predetermined shape. The nanostructure backbone may be a DNA-origami. These DNA origami structures may range in size from a few nanometres into the micron range. For the fabrication of such DNA origami-based structures longer DNA molecules (scaffold strands) are folded at precisely identified positions by so called staple strands. The DNA origami may be designed to provide a self-assembly nanostructure backbone of a particular predetermined shape. This enables an easy and reproducible synthesis and assembly of the backbone. Staple strands may be position-selectively functionalised. The positional resolution in this case is limited by the size of a nucleotide, which is in the range of a nanometre or below. This has been exploited in the prior art to generate fluorescent standards, wherein fluorescent dyes are connected to precisely located bands on the DNA origami. These standards are known as "nanoruler" and are used for the calibration of imaging systems like confocal or super resolution microscopes (e.g. STED), for example, as disclosed by US2014/0057805 A1.

The DNA origami provides a scaffold for the labels. Preferably, the DNA origami structure comprises at least one scaffold strand and multiple staple strands, wherein the staple strands are complementary to at least parts of the scaffold strand and configured to bring the scaffold strand into a predetermined conformation. In particular, the strands are oligonucleotides. This enables generating nanostructure backbones with predetermined two- or three-dimensional shapes that can self-assemble. Further, this enables the site-specific placement of attachment sites on the backbone.

The attachment sites being unique nucleic acid sequences, preferably of the staple strands. Preferably, the labels may be attached to staple strands of the nanostructure backbone at predetermined attachment sites. Since the staple strands are located at predetermined positions the positions of the attachment sites may equally be predetermined. Thus, the attachment site is a unique oligonucleotide sequence complementary to the attachment oligonucleotide portion of one label.

Preferably, the largest spatial extent of the nanostructure backbone is in a range from 10 nm to 10000 nm, preferably in a range from 0.1 µm to 5 µm. This enables a particularly compact storage device and allows an optical readout, for example with a light microscope.

Preferably, the attachment sites are spaced apart from each other in a range from 1 nm to 2000 nm, preferably in a range from 200 nm to 1000 nm. This enables a particularly dense arrangement of labels on the nanostructure backbone. The spacing between the attachment sites may be chosen depending on the resolving power of a readout device used to read out the labels. Particularly preferable ranges may correspond to the lateral resolution achievable with different microscopic modalities such as for example single molecule localization microscopy (1 nm to 25 nm), structured illumination and STED microscopy (50 nm to 100 nm), high NA (numerical aperture) light microscopy (around 200 nm), and low NA light microscopy (around 500 nm). Importantly, the labels may be distanced from each other such that the readout device can resolve the labels individually.

Preferably, the dye is a fluorophore. Preferably each label has fluorophore(s) with different characteristics, such as excitation/emission wavelength, to enable generating a larger number of different labels. According to a preferred embodiment, the dye is a combination of dyes as described in the patent application with the application number PCT/EP2021/073819.

Preferably, the nanostructure backbone extends linearly in one dimension and the first orientation indicator and the second orientation indicator are spaced apart from each other, or arranged on opposite ends of the nanostructure backbone. This enables determining the orientation of the storage device and/or of the oligonucleotide nanostructure backbone. The orientation indicators may comprise fluorescent dyes, for example. In particular, the first orientation indicator and the second orientation indicator have different properties, such as excitation wavelength, fluorescence emission wavelength, and/or fluorescence lifetime.

Preferably, the nanostructure backbone extends in two dimensions or three dimensions and the nanostructure backbone comprises at least a third orientation indicator. This enables determining the orientation of the storage device as well as providing a two-dimensional or three-dimensional storing device enabling to store additional positional information in two or three dimensions.

Preferably, the labels further comprise an encoding oligonucleotide portion configured to encode characteristics of the at least one dye. This enables reading out the storage device not only visually, but also by sequencing of at least the labels of the storage device.

Preferably, the labels comprise primer sequences configured to enable sequencing of the attachment portion and/or the encoding portion, preferably, all labels comprise the same primer sequences. This enables particular efficient sequencing of the portions.

Preferably, each label comprises a cleavage site configured to separate the dye from the label. The cleavage site may allow enzymatic, temperature, or light induced cleavage. This enables efficient removal of the dye from portions of the label, particularly prior to sequencing the portions.

In a further aspect, a method for storing and accessing data is provided, comprising the following steps: generating or providing at least one data storage device, in particular having the characteristics as described above, including selecting a label with at least one dye based on the data to be stored at a particular one of the attachment sites and based on a predetermined set of rules, in particular, the set of rules associating the dye to a particular data; generating an optical readout of the data storage device; determining in the optical readout the at least one dye of the label for the particular one of the attachment sites; and retrieving the data based on the predetermined set of rules, in particular, the set of rules may include information about the particular one of the attachment sites.

The optical readout may be an image-based readout, in particular a 2D image or an image stack or a 3D image, which may be acquired using a microscope like a point-scanning confocal or a camera-based/widefield imaging system or for example a spinning disk microscope, a light sheet fluorescence microscope, a light field microscope, a stereomicroscope. Further the optical readout may be non-image-based readouts for example in a cytometer or a flow-through based readout device with at least one point detector or a line detector. A readout may consist of a discrete readout, for example a single acquisition of an emission spectrum or image stack, a readout may be a readout data stream, for example in a point-scanning confocal or cytometer, which is substantially continuous. Further a readout may be a sequence of images for example a spectral or hyperspectral image stack, wherein in each image fluorescence emission of different wavelength bands is recorded.

The optical readout may be generated by a readout device used to perform fluorescence multi-colour reading or imaging. The readout device typically includes at least one excitation light source, a detection system including at least one detection channel and may further contain filters and/or dispersive optical elements such as prisms and/or gratings to route excitation light to the device and/or to route emission light from the device onto one or more detectors or onto an appropriate area of a detector. The detection system may comprise several detection channels, may be a spectral detector detecting multiple bands of the spectrum in parallel, or a hyperspectral detector detecting a contiguous part of the spectrum. The detection system contains at least one detector, which may be a point-detector (e.g. a photomultiplier, an avalanche diode, a hybrid detector), an array-detector, a camera, hyperspectral camera. The detection system may record intensities per channel as is typically the case in cytometers or may be an imaging detection system that records images as in the case of plate readers or microscopes. A readout device with one detector channel, for example a camera or a photomultiplier, may generate readouts with multiple detection channels using, for example, different excitation and emission bands.

Preferably, the predetermined set of rules is stored for accessing the data and information about the particular one of the attachment sites. This enables efficient retrieval of the data from the storage device. For secure storage of data with the data storage device, the predetermined set of rules may be kept secret. Alternatively, the predetermined set of rules may be public knowledge.

In a further aspect, a plurality of labels is provided, for attaching to a plurality of attachment sites of an oligonucleotide nanostructure backbone, each label comprises at least one dye and an attachment oligonucleotide portion configured to attach the label to one of the attachment sites of the oligonucleotide nanostructure backbone, and the attachment oligonucleotide portion of each label comprises a unique oligonucleotide sequence configured to bind to a complementary sequence of one of the attachment sites of the oligonucleotide nanostructure backbone in order to generate a data storage device according to one of the preceding claims by attaching at least one of the labels to one of the attachment sites of the oligonucleotide nanostructure backbone.

In another aspect, at least one oligonucleotide nanostructure backbone is provided comprising: a plurality of attachment sites at predetermined positions and at least a first orientation indicator and a second orientation indicator, the attachment sites of the oligonucleotide nanostructure backbone are configured to attach a plurality of labels, each label comprising at least one dye and an attachment oligonucleotide portion configured to attach the label to one of the attachment sites of the oligonucleotide nanostructure backbone, and the attachment oligonucleotide portion of each label comprises a unique oligonucleotide sequence configured to bind to a complementary sequence of one of the attachment sites of the oligonucleotide nanostructure backbone in order to generate a data storage device according to one of the preceding claims by attaching at least one of the labels to one of the attachment sites of the oligonucleotide nanostructure backbone.

In a further aspect, a database is provided comprising information about the predetermined set of rules, in particular, corresponding to at least one of the following: at least one oligonucleotide nanostructure backbone; at least a first orientation indicator and a second orientation indicator of the at least one oligonucleotide nanostructure backbone; a plurality of attachment sites of the at least one oligonucleotide nanostructure backbone; predetermined positions of the plurality of attachment sites of the at least one oligonucleotide nanostructure backbone; a plurality of labels; at least one dye of each label; an attachment oligonucleotide portion of each label, in particular, being configured to attach the label to one of the attachment sites of the oligonucleotide nanostructure backbone; and which label is attached to which attachment site of the oligonucleotide nanostructure backbone.

### Short Description of the Figures:

Hereinafter, specific embodiments are described referring to the drawings, wherein:
- Fig. 1: shows several nanostructure backbones with orientation indicators,
- Fig. 2: shows a label for attachment to attachment sites of the nanostructure backbones,
- Fig. 3: shows a variety of rod-shaped data storage devices,
- Fig. 4: shows details of a rod-shaped data storage device,
- Fig. 5: shows a cube-shaped data storage device,
- Fig. 6: shows the elements of a data storage device for storing data, and
- Fig. 7: shows a flow chart of a method for storing and retrieving data.

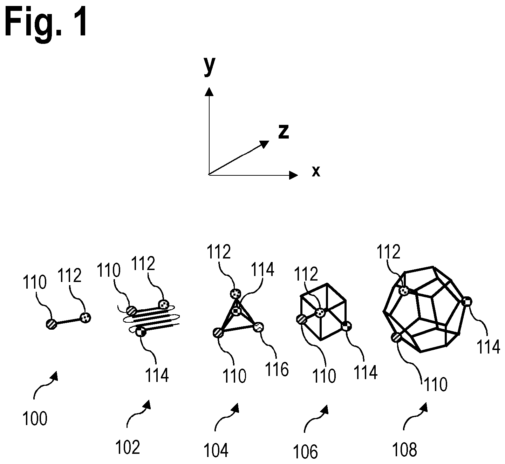

Figure 1 shows schematically oligonucleotide nanostructure backbones 100, 102, 104, 106, 108 with different geometries. Generally, the nanostructure backbones 100, 102, 104, 106, 108 comprise nucleic acids. In particular, the nanostructure backbones 100, 102, 104, 106, 108 are DNA-origami based, which allows generating predetermined, stable two- and three-dimensional shapes. Further, this allows generating a plurality of attachment sites at predetermined positions along the nanostructure backbones 100, 102, 104, 106, 108. The attachment sites are stretches of oligonucleotide, that are unique and allow the hybridisation of complementary oligonucleotides, for example to attach labels to the nanostructure backbones 100, 102, 104, 106, 108 at these predetermined positions.

Nanostructure backbone 100 is linear or rod-like. It comprises a first orientation indicator 110 and a second orientation indicator 112. The orientation indicators 110, 112 may be used to determine the orientation, directionality or polarity of the nanostructure backbone 100. The orientation indicators 110, 112 may comprise a dye, in particular a fluorescent dye, such as fluorescein or a fluorescent protein. In addition, the dye of the first orientation indicator 110 has different characteristics than the dye of the second orientation indicator 112. The characteristics may include fluorescent emission characteristics, excitation characteristics or lifetime characteristics. This enables differentiating between the first and the second orientation indicators 110, 112 in an optical readout of the nanostructure 100, for example generated by a microscope, a cytometer, or an imaging cytometer. The orientation indicators 110, 112 are arranged spaced apart from each other. Preferably each orientation indicator 110, 112 is arranged on the backbone 100 at opposite ends. Thus, the first and second orientation indicators 110, 112 enable differentiating between a first end and a second end of the backbone 100. Ultimately, this enables determining the orientation, directionality or polarity of the backbone 100, for example from the first orientation indicator 110 on the first end to the second orientation indicator 112 on the second end.

The nanostructure 102 is sheet-like, which may be a large linear DNA molecule or an assembly of multiple DNA molecules. Sheet-like nanostructures may increase the number of available attachment sites substantially. In order to be able to determine the orientation of the nanostructure 102, a third orientation indicator 114 is provided.

Further geometries are possible, for example, the tetrahedral nanostructure 104, the cubic nanostructure 106, or the polyhedral nanostructure 108. These may comprise a fourth orientation indicator 116 in order to determine their orientation.

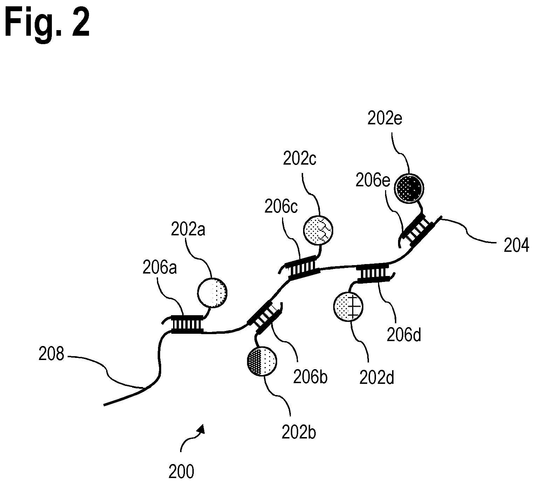

Figure 2 shows schematically a label 200 for attachment to the attachment sites of the nanostructure backbones 100, 102, 104, 106, 108. The label 200 comprises several fluorescent dyes 202a, 202b, 202c, 202d, 202e. The fluorescent dyes 202a to 202e may differ in their fluorescent properties, for example, excitation wavelengths, emission wavelengths and fluorescent lifetime characteristics. Preferably, the dyes 202a to 202e of the label 200 may be individually identified in a read-out in particular of the label 200. Depending on the number and type of dyes being used when generating the label 200, a certain number of unique labels can be generated. Typically, the number of different dyes used in total may be in the range of 5-50, for example. For 20 dyes and when using 5 dyes for each label it is easily possible to generate a set of labels with 15,504 unique labels.

The dyes 202a to 202e are individually attached to a label support 204. The label support 204 may be an oligonucleotide and each of the dyes 202a to 202e may be specifically attached to the label support 204 via a unique hybridisation part 206a, 206b, 206c, 206d, 206e. Moreover, the one end 208 of the label support 204 may be specifically attached to the nanostructure backbones 100 to 108, as described in more detail below. The orientation indicators 110, 112, 114, 116, preferably have the same structure as described for the label 200. Thus, the dye can in particular be a combination of dyes as described in the patent application with the application number PCT/EP2021/073819.

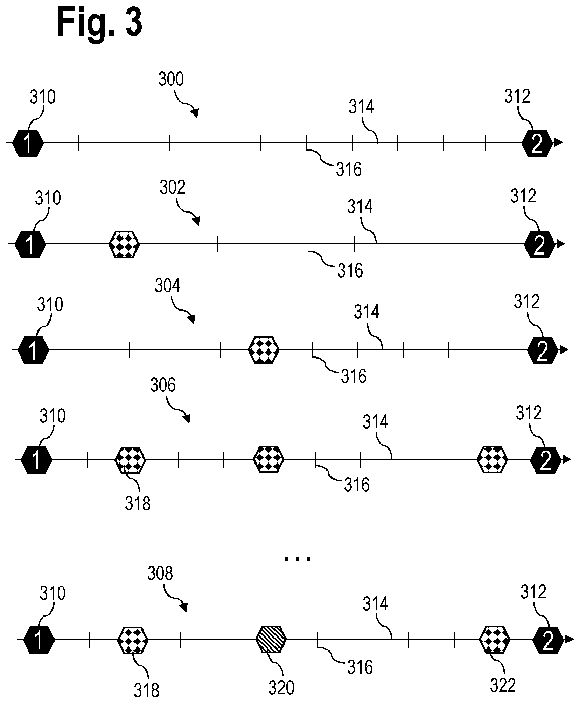

Figure 3 shows schematically a variety of rod-shaped data storage devices 300, 302, 304, 306, 308. The data storage devices 300 to 308 each comprise a first orientation indicator 310 and a second orientation indicator 312, at a first attachment site and a second attachment site of the nanostructure backbone 314, respectively. In addition, there are ten further attachment sites, one of which is indicated by reference sign 316. In case of the data storage devices 308, labels 318, 320, 322 are attached at three further attachment sites 316. The attachment sites 316 of each nanostructure backbone 314 is unique such that the labels 318, 320, 322 are specifically attachable to a particular attachment site 316. The data storage devices 300, 302, 304, 306, 308 are preferably between 1 to 2 µm in length.

The orientation indicators 310, 312 generate a relative coordinate system for the data storage devices 300, 302, 304, 306, 308, on which each attachment site 316 may be placed. For example, each attachment site 316 may be assigned an index n with n=1, 2, 3, ..., based on the unique location of the respective attachment site 316. Thus, the different data storage devices 300 to 308 can all be identified and/or distinguished visually, due to their use of labels with differing properties and/or the labels being attached at different, distinguishable attachment sites 316 along the nanostructure backbone 314.

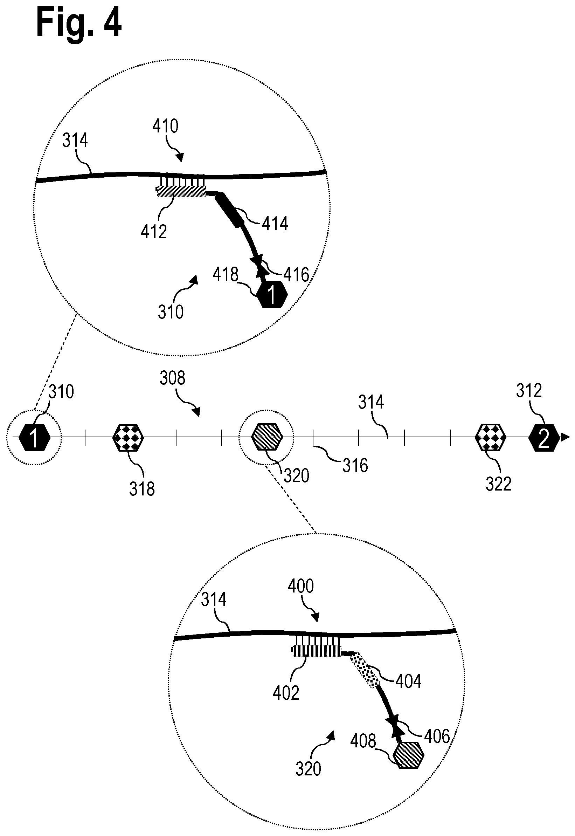

Figure 4 shows schematically details of the rod-shaped data storage device 308, in particular, the label 320 and its attachment to the nanostructure backbone 314. The label 320 is attached to the nanostructure backbone 314 at a particular attachment site 400 of the nanostructure backbone 314. This attachment site 400 has a unique oligonucleotide sequence that allows hybridisation of a complementary attachment oligonucleotide portion 402 of the label 320. Thus, each attachment site of the data storage device 308 has a unique oligonucleotide sequence enabling to specifically target labels for each one of the attachment sites. Thus, each label has a unique sequence that hybridises to a particular one of the attachment sites of a nanostructure backbone (e.g. the attachment portion 402).

Optionally in addition, the label 320 may comprise an encoding oligonucleotide portion 404. The encoding portion 404 is an oligonucleotide sequence that is unique to the fluorescent dye or dyes 408 of the label 320. This means that the dyes of a particular label may be identified by the sequence of their encoding portion. To facilitate sequencing the label 320 may also comprises a cleavage site 406 for removing the dye 408 from the encoding portion 404 and the attachment portion 402 prior to sequencing. This allows not only reading the label and the data storage device visually, but also by sequencing.

The first and second orientation indicators 310, 312 are similarly constructed. For example, the orientation indicator 310 is attached to an attachment site 410 of the nanostructure backbone 314 by a unique complementary attachment oligonucleotide portion 412.

Optionally, the orientation indictor 310 may further comprise an encoding portion 414, that is unique to the dye or dyes 418 of the orientation indicator. The dyes 418 may be removed from the encoding portion 414 and the attachment portion 412 by cleaving an optional cleavage site 416, as explained for label 320.

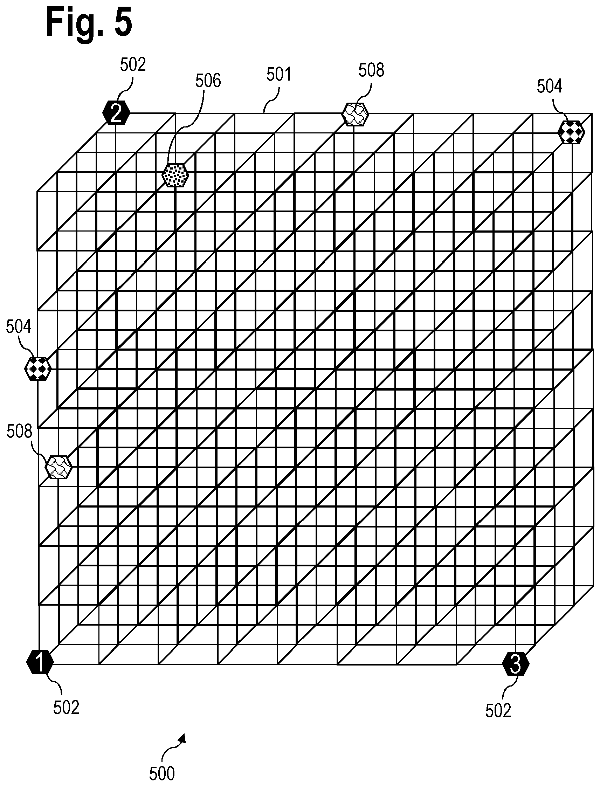

Figure 5 shows schematically an example of a cube-shaped data storage device 500 with a three-dimensional array nanostructure backbone 501. The data storage device 500 comprises three orientation indicators 502. Further, the data storage device 500 comprises a set of different labels 504, 506, 508. Each label 504, 506, 508 is attached at a particular attachment site of the data storage device 500. The attachment sites may, for example, be at the corners or edges of the array of the backbone 501. The labels 504, 506, 508 differ in their fluorescent properties, such as fluorescent lifetime, emission wavelength and excitation wavelength.

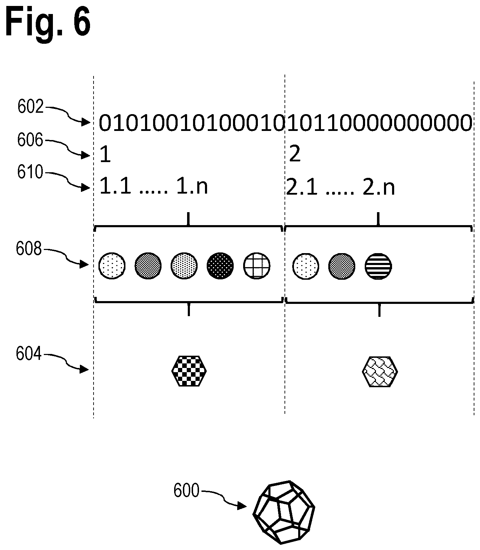

Thus, similarly to the data storage devices 300 to 308 in Figure 3, the data storage device 500 may contain information in the particular placement of labels on the attachment sites of the backbone 501. This information may be readout visually. In comparison to the rod-shaped data storage devices 300 to 308, the cube-shaped data storage device 500 provides for around 360 attachment sites for labels, which increases the number of possible combinations of labels and their position on the backbone 501. This increases the possible amount of information that may be stored in the data storage device 500. A further parameter to increase storage capacity is the number of different dyes that may be used to generate the labels. By these means, a data storage density of about 1 to 5 Tbit/inch² can be achieved. A particular example of data storage with such a data storage device is explained with Figure 6. The physical size of the cube-shaped storage device 500 is in the range of 2.5 to 10 µm per side of the cube.

Figure 6 shows schematically how elements of a data storage device 600 may be used to store data on the data storage device and retrieve it. That data is shown in Figure 6 as a sequence of binary data 602. The binary data 602 may be encoded in several labels 604, exemplarily in Figure 6 two labels 604, attachable to attachment sites of a nanostructure backbone of data storage device 600. Thus, the binary data 602 is stored over several labels 604 and distributed accordingly. Each label 604 is assigned a specific attachment site 606 of the backbone of the storage device 600, that means, that each label has a particular attachment oligonucleotide sequence that enables hybridisation with that specific attachment site 606. Since each label 604 may comprise several dyes 608, each attachment site 606 or label 604 may store a corresponding amount of the data 602. For example, if 10 dyes 608 are used, each label 604 or each attachment site 606 may hold 10 bits. Thus, each dye 608 stores a particular part of the data 602. Specifically, each dye 608 has a corresponding index 610, that identifies the attachment site 606 and the particular part of the data 602 that is stored in that dye 608. For example, the index 610 of 1.1 identifies the dye 608 that encodes the first bit of data 602 at the first attachment site 606. Specific characteristics of the dyes 608, such as their fluorescent lifetime, emission/excitation wavelengths, may be defined as representing a particular state (e.g. 0 or 1) of a particular bit of the binary data 602. Alternatively, the presence of a particular dye 608 at a particular index 610 may represent a particular state (e.g. 0 or 1) of the particular bit of data 602 at the particular index 610. Alternatively or additionally, any other representation could be used. For instance, an octal, a decimal, or a hexadecimal representation, in particular depending on the number of different dyes in a combination of dyes as described in the patent application with the application number PCT/EP2021/073819. If e.g. eight different dyes are used for one combination of dyes, an octal representation of the information could be used. The higher the number of different dyes is used for one combination of dyes 604, the less attachment sites 606 are necessary in order to encode the same information. Furthermore, it might be possible to even apply different representations based on different numbers of different dyes in the combination of dyes, if the dyes 604 of e.g. an octal representation are only applied to a particular kind of oligonucleotide nanostructure backbone being identifiable by the at least first, second and/or any further orientation indicator. Moreover, since the dyes 608 may differ in more than one characteristic, the dyes 608 may represent more than two (binary) states.

In order to store the data 602 in the storage device 600, the storage device 600 has to be generated based on the data 602 to be saved and based on a set of rules. The set of rules may be predetermined before generation of the device 600 and comprises the information discussed in the above paragraph. Specifically, at least the following information is necessary for storing the data 602: the layout of the nanostructure backbone of the device 600, in particular the numbering of the attachment sites 606 on the backbone or in other words the sequence of attachment sites 606 in which the data is encoded, the assignment of a particular dye 608 or characteristic of the dye 608 to a state (e.g. 0 or 1), and which dye 608 of a label 604 corresponds to which bit of the data 602 at a particular attachment site 606 (this being encoded in the index 610). Subsets of this information may be used as private keys and public keys, for example, for asymmetric cryptography. Alternatively, both keys may be held private.

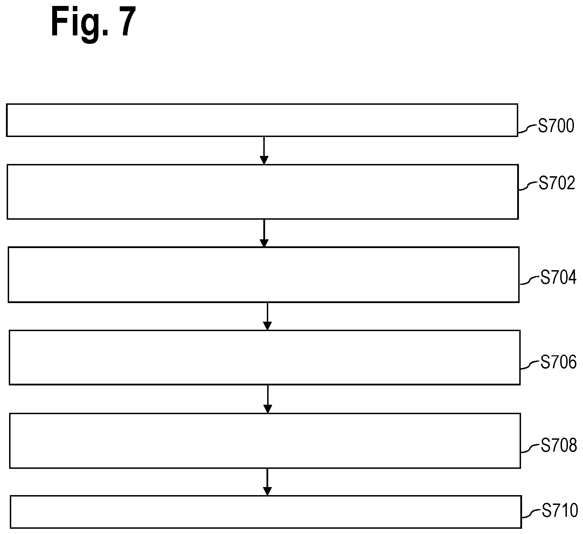

Figure 7 shows a flow chart of a method for storing and accessing the data 602 in the storage device 600. The method starts in step S700. In a first step S702, the data storage device 600 is generated from a nanostructure backbone and labels based on a set of rules described with Figure 6. This stores the data 602 in the device 600.

In step S704, an optical readout of the device 600 is generated in order to retrieve or access the data 602 stored in the device 600.

Alternatively, in case the device comprises encoding oligonucleotide portions, as described above, the dyes of a particular label may be identified by the sequence of their encoding portion. This means that in this case, the device may be readout by sequencing the device.

In step S706, the optical readout generated in step S704 is analysed to determine for each attachment site 606 the dyes 608 of the respective labels 604.

In step S708, the data 602 is retrieved from the information determined in step S706 based on the set of rules used in step S702. To that end, the set of rules may be stored, in order to facilitate data retrieval, and/or the set of rules may be kept secret, in order to secure the data 602 stored in the device 600. The method ends in step S710.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100, 102, 104, 106, 108, 314, 501: Nanostructure backbone
- 110, 112, 114, 310, 312, 502: Orientation indicator
- 200, 318, 320, 322, 504, 506, 508, 604: Label
- 202a, 202b, 202c, 202d, 202e, 408, 418, 608: Fluorescent dye
- 204: Label support
- 206a, 206b, 206c, 206d, 206e: Hybridisation part
- 208: End of label support
- 406, 416: Cleavage site
- 300, 302, 304, 306, 308, 500, 600: Data storage device
- 316, 400, 410, 606: Attachment site
- 402, 412: Attachment oligonucleotide portion
- 404, 414: Encoding oligonucleotide portion
- 602: Binary data
- 610: Index

## Claims

1. A data storage device (300, 302, 304, 306, 308, 500, 600) comprising:
an oligonucleotide nanostructure backbone (100, 102, 104, 106, 108, 314, 501) with a plurality of attachment sites (316, 400, 410, 606) at predetermined positions,
a plurality of labels (200, 318, 320, 322, 504, 506, 508, 604) attached to the attachment sites (316, 400, 410, 606),
at least a first orientation indicator and a second orientation indicator (110, 112, 114, 310, 312, 502), each attached to the backbone for visually determining the orientation of the data storage device,
wherein each label (200, 318, 320, 322, 504, 506, 508, 604) comprises at least one dye (202a, 202b, 202c, 202d, 202e, 408, 418, 608), and an attachment oligonucleotide portion (402, 412) attached to one of the attachment sites (316, 400, 410, 606), and
wherein the attachment oligonucleotide portion (402, 412) of each label (200, 318, 320, 322, 504, 506, 508, 604) comprises a unique oligonucleotide sequence configured to bind to a complementary sequence of one of the attachment sites (316, 400, 410, 606).

2. The data storage device according to claim 1, wherein the nanostructure backbone (100, 102, 104, 106, 108, 314, 501) comprises scaffold strands, staple strands configured to bind to the scaffold strands at predetermined positions to fold the scaffold strand into a predetermined shape.

3. The data storage device according to claim 2, wherein the staple strands comprise the attachment sites (316, 400, 410, 606).

4. The data storage device according to one of the preceding claims, wherein the largest spatial extent of the nanostructure backbone (100, 102, 104, 106, 108, 314, 501) is in a range from 10 nm to 10000 nm, preferably in a range from 0.1 µm to 5 µm.

5. The data storage device according to one of the preceding claims, where the attachment sites (316, 400, 410, 606) are spaced apart from each other in a range from 1 nm to 2000 nm, preferably in a range from 200 nm to 1000 nm.

6. The data storage device according to one of the preceding claims, wherein the dye (202a, 202b, 202c, 202d, 202e, 408, 418, 608) is a fluorophore.

7. The data storage device according to one of the preceding claims, wherein the nanostructure backbone (100, 102, 104, 106, 108, 314, 501) extends linearly in one dimension and the first orientation indicator and the second orientation indicator are spaced apart from each other, or arranged on opposite ends of the nanostructure backbone (100, 102, 104, 106, 108, 314, 501).

8. The data storage device according to one of the preceding claims 1 to 6, wherein the nanostructure backbone (100, 102, 104, 106, 108, 314, 501) extends in two dimensions or three dimensions and the nanostructure backbone (100, 102, 104, 106, 108, 314, 501) comprises at least a third orientation indicator.

9. The data storage device according to one of the preceding claims, wherein the labels (200, 318, 320, 322, 504, 506, 508, 604) further comprise an encoding oligonucleotide portion (404, 414) configured to encode characteristics of the at least one dye (202a, 202b, 202c, 202d, 202e, 408, 418, 608).

10. The data storage device according to claim 9, wherein the labels (200, 318, 320, 322, 504, 506, 508, 604) comprises primer sequences configured to enable sequencing of at least the encoding portion, preferably, all labels (200, 318, 320, 322, 504, 506, 508, 604) comprise the same primers sequences.

11. A method for storing and accessing data comprising the following steps:
generating or providing at least one data storage device (300, 302, 304, 306, 308, 500, 600) according to one of the preceding claims, including selecting a label (200, 318, 320, 322, 504, 506, 508, 604) with at least one dye (202a, 202b, 202c, 202d, 202e, 408, 418, 608) based on the data to be stored at a particular one of the attachment sites (316, 400, 410, 606) and based on a predetermined set of rules,
generating an optical readout of the data storage device,
determining in the optical readout the at least one dye (202a, 202b, 202c, 202d, 202e, 408, 418, 608) of the label (200, 318, 320, 322, 504, 506, 508, 604) for the particular one of the attachment sites (316, 400, 410, 606), and
retrieving the data based on the predetermined set of rules.

## Patentansprüche

1. Datenspeichervorrichtung (300, 302, 304, 306, 308, 500, 600), umfassend: ein Oligonukleotid-Nanostrukturrückgrat (100, 102, 104, 106, 108, 314, 501) mit einer Vielzahl von Anheftungsstellen (316, 400, 410, 606) an vorbestimmten Positionen,
eine Vielzahl von Markierungen (200, 318, 320, 322, 504, 506, 508, 604), die an den Anheftungsstellen (316, 400, 410, 606) angeheftet sind, mindestens einen ersten Orientierungsindikator und einen zweiten Orientierungsindikator (110, 112, 114, 310, 312, 502), die jeweils an das Rückgrat angeheftet sind, um die Orientierung der Datenspeichervorrichtung visuell zu bestimmen,
wobei jede Markierung (200, 318, 320, 322, 504, 506, 508, 604) mindestens einen Farbstoff (202a, 202b, 202c, 202d, 202e, 408, 418, 608) und einen Anheftungsoligonukleotid-Abschnitt (402, 412) umfasst, der an eine der Anheftungsstellen (316, 400, 410, 606) angeheftet ist, und
wobei der Anheftungsoligonukleotid-Abschnitt (402, 412) jeder Markierung (200, 318, 320, 322, 504, 506, 508, 604) eine eindeutige Oligonukleotidsequenz umfasst, die ausgebildet ist, an eine komplementäre Sequenz einer der Anheftungsstellen (316, 400, 410, 606) zu binden.

2. Datenspeichervorrichtung nach Anspruch 1, wobei das Nanostrukturrückgrat (100, 102, 104, 106, 108, 314, 501) Gerüststränge, Klammerstränge umfasst, die ausgebildet sind, an die Gerüststränge an vorbestimmten Positionen zu binden, um den Gerüststrang in eine vorbestimmte Form zu falten.

3. Datenspeichervorrichtung nach Anspruch 2, wobei die Klammerstränge die Anheftungsstellen (316, 400, 410, 606) umfassen.

4. Datenspeichervorrichtung nach einem der vorstehenden Ansprüche, wobei die größte räumliche Ausdehnung des Nanostrukturrückgrats (100, 102, 104, 106, 108, 314, 501) in einem Bereich von 10 nm bis 10000 nm liegt, bevorzugt in einem Bereich von 0,1 µm bis 5 µm.

5. Datenspeichervorrichtung nach einem der vorstehenden Ansprüche, wobei die Anheftungsstellen (316, 400, 410, 606) in einem Bereich von 1 nm bis 2000 nm, bevorzugt in einem Bereich von 200 nm bis 1000 nm, voneinander beabstandet sind.

6. Datenspeichervorrichtung nach einem der vorstehenden Ansprüche, wobei der Farbstoff (202a, 202b, 202c, 202d, 202e, 408, 418, 608) ein Fluorophor ist.

7. Datenspeichervorrichtung nach einem der vorstehenden Ansprüche, wobei sich das Nanostrukturrückgrat (100, 102, 104, 106, 108, 314, 501) in einer Abmessung linear erstreckt und der erste Orientierungsindikator und der zweite Orientierungsindikator voneinander beabstandet sind oder an entgegengesetzten Enden des Nanostrukturrückgrats (100, 102, 104, 106, 108, 314, 501) angeordnet sind.

8. Datenspeichervorrichtung nach einem der vorstehenden Ansprüche 1 bis 6, wobei sich das Nanostrukturrückgrat (100, 102, 104, 106, 108, 314, 501) in zwei Abmessungen oder drei Abmessungen erstreckt und das Nanostrukturrückgrat (100, 102, 104, 106, 108, 314, 501) mindestens einen dritten Orientierungsindikator umfasst.

9. Datenspeichervorrichtung nach einem der vorstehenden Ansprüche, wobei die Markierungen (200, 318, 320, 322, 504, 506, 508, 604) ferner einen Kodieroligonukleotid-Abschnitt (404, 414) umfassen, der ausgebildet ist, Eigenschaften des mindestens einen Farbstoffs (202a, 202b, 202c, 202d, 202e, 408, 418, 608) zu kodieren.

10. Datenspeichervorrichtung nach Anspruch 9, wobei die Markierungen (200, 318, 320, 322, 504, 506, 508, 604) Primersequenzen umfassen, die ausgebildet sind, eine Sequenzierung mindestens des Kodierabschnitts zu ermöglichen, bevorzugt umfassen alle Markierungen (200, 318, 320, 322, 504, 506, 508, 604) dieselben Primersequenzen.

11. Verfahren zum Speichern und Zugreifen auf Daten, umfassend die folgenden Schritte:
Erzeugen oder Bereitstellen von mindestens einer Datenspeichervorrichtung (300, 302, 304, 306, 308, 500, 600) nach einem der vorstehenden Ansprüche, einschließlich Auswählens einer Markierung (200, 318, 320, 322, 504, 506, 508, 604) mit mindestens einem Farbstoff (202a, 202b, 202c, 202d, 202e, 408, 418, 608) basierend auf den in einer bestimmten der Anheftungsstellen (316, 400, 410, 606) zu speichernden Daten und basierend auf einem vorbestimmten Regelsatz,
Erzeugen einer optischen Auslesung der Datenspeichervorrichtung,
Bestimmen in der optischen Auslesung des mindestens einen Farbstoffs (202a, 202b, 202c, 202d, 202e, 408, 418, 608) der Markierung (200, 318, 320, 322, 504, 506, 508, 604) für die bestimmte der Anheftungsstellen (316, 400, 410, 606), und
Abrufen der Daten basierend auf dem vorbestimmten Regelsatz.

## Revendications

1. Dispositif de stockage de données (300, 302, 304, 306, 308, 500, 600) comprenant : une ossature de nanostructure oligonucléotidique (100, 102, 104, 106, 108, 314, 501) avec une pluralité de sites d'attache (316, 400, 410, 606) à des positions prédéterminées,
une pluralité d'étiquettes (200, 318, 320, 322, 504, 506, 508, 604) attachées aux sites d'attache (316, 400, 410, 606), au moins un premier indicateur d'orientation et un deuxième indicateur d'orientation (110, 112, 114, 310, 312, 502), chacun étant attaché à l'ossature pour déterminer visuellement l'orientation du dispositif de stockage de données,
dans lequel chaque étiquette (200, 318, 320, 322, 504, 506, 508, 604) comprend au moins un colorant (202a, 202b, 202c, 202d, 202e, 408, 418, 608), et une portion oligonucléotidique d'attache (402, 412) attachée à l'un des sites d'attache (316, 400, 410, 606), et
dans lequel la portion oligonucléotidique d'attache (402, 412) de chaque étiquette (200, 318, 320, 322, 504, 506, 508, 604) comprend une séquence oligonucléotidique unique configurée pour se lier à une séquence complémentaire de l'un des sites d'attache (316, 400, 410, 606).

2. Dispositif de stockage de données selon la revendication 1, dans lequel l'ossature de nanostructure oligonucléotidique (100, 102, 104, 106, 108, 314, 501) comprend des brins d'échafaudage, des brins d'agrafage configurés pour se lier aux brins d'échafaudage à des positions prédéterminées pour plier le brin d'échafaudage en une forme prédéterminée.

3. Dispositif de stockage de données selon la revendication 2, dans lequel les brins d'agrafage comprennent les sites d'attache (316, 400, 410, 606).

4. Dispositif de stockage de données selon l'une des revendications précédentes, dans lequel la plus grande extension spatiale de l'ossature de nanostructure oligonucléotidique (100, 102, 104, 106, 108, 314, 501) est dans une plage allant de 10 nm à 10 000 nm, de préférence dans une plage allant de 0,1 µm à 5 µm.

5. Dispositif de stockage de données selon l'une des revendications précédentes, où les sites d'attache (316, 400, 410, 606) sont espacés les uns des autres dans une plage allant de 1 nm à 2 000 nm, de préférence dans une plage allant de 200 nm à 1 000 nm.

6. Dispositif de stockage de données selon l'une des revendications précédentes, dans lequel le colorant (202a, 202b, 202c, 202d, 202e, 408, 418, 608) est un fluorophore.

7. Dispositif de stockage de données selon l'une des revendications précédentes, dans lequel l'ossature de nanostructure oligonucléotidique (100, 102, 104, 106, 108, 314, 501) s'étend linéairement dans une dimension et le premier indicateur d'orientation et le deuxième indicateur d'orientation sont espacés l'un de l'autre, ou disposés sur des extrémités opposées de l'ossature de nanostructure oligonucléotidique (100, 102, 104, 106, 108, 314, 501).

8. Dispositif de stockage de données selon l'une des revendications précédentes 1 à 6, dans lequel l'ossature de nanostructure oligonucléotidique (100, 102, 104, 106, 108, 314, 501) s'étend dans deux dimensions ou trois dimensions et l'ossature de nanostructure oligonucléotidique (100, 102, 104, 106, 108, 314, 501) comprend au moins un troisième indicateur d'orientation.

9. Dispositif de stockage de données selon l'une des revendications précédentes, dans lequel les étiquettes (200, 318, 320, 322, 504, 506, 508, 604) comprennent en outre une portion oligonucléotidique de codage (404, 414) configurée pour coder des caractéristiques d'au moins un colorant (202a, 202b, 202c, 202d, 202e, 408, 418, 608).

10. Dispositif de stockage de données selon la revendication 9, dans lequel les étiquettes (200, 318, 320, 322, 504, 506, 508, 604) comprennent des séquences d'amorce configurées pour permettre le séquençage d'au moins la portion de codage, de préférence, toutes les étiquettes (200, 318, 320, 322, 504, 506, 508, 604) comprennent les mêmes séquences d'amorce.

11. Procédé de stockage et d'accès à des données comprenant les étapes suivantes :
génération ou fourniture d'au moins un dispositif de stockage de données (300, 302, 304, 306, 308, 500, 600) selon l'une des revendications précédentes, incluant la sélection d'une étiquette (200, 318, 320, 322, 504, 506, 508, 604) avec au moins un colorant (202a, 202b, 202c, 202d, 202e, 408, 418, 608) sur la base des données devant être stockées au niveau d'un site particulier des sites d'attache (316, 400, 410, 606) et sur la base d'un ensemble prédéterminé de règles,
génération d'une lecture optique du dispositif de stockage de données,
détermination dans la lecture optique d'au moins un colorant (202a, 202b, 202c, 202d, 202e, 408, 418, 608) de l'étiquette (200, 318, 320, 322, 504, 506, 508, 604) pour le site particulier des sites d'attache (316, 400, 410, 606), et
récupération des données sur la base de l'ensemble prédéterminé de règles.
